# NOUVEAU FASCICULE DE BREVET EUROPEEN

(11) **EP 1 051 174 B2**
(45) Date de publication et mention de la décision concernant l'opposition: **04.07.2012**
(45) Mention de la délivrance du brevet: 05.02.2003
(21) Numéro de dépôt: 98942718.2
(22) Date de dépôt: 10.08.1998
(51) Int. Cl.: A61K 31/44, A61K 9/54

(54) **MICROGRANULES D'OMEPRAZOLE GASTROPROTEGES, PROCEDE D'OBTENTION ET PREPARATIONS PHARMACEUTIQUES**
GEGEN MAGENSAFT GESCHÜTZTE OMEPRAZOL-MIKROGRANÜLEN, HERSTELLUNGSVERFAHREN UND PHARMAZEUTISCHE ZUBEREITUNGEN
GASTROPROTECTED OMERPRAZOLE MICROGRANULES, METHOD FOR OBTAINING SAME AND PHARMACEUTICAL PREPARATIONS

(30) Priorité: 30.01.1998 FR 9801098
(43) Date de publication de la demande: 15.11.2000
(73) Titulaire: ETHYPHARM, E-24004 Madrid (ES)
(72) Inventeur: DEBREGEAS, Patrice, F-75007 Paris (FR); LEDUC, Gérard, F-45330 Malesherbes (FR); OURY, Pascal, F-75005 Paris (FR); SUPLIE, Pascal, F-27400 Montaure (FR)
(74) Mandataire: Texier, Christian
(86) Numéro de dépôt international: PCT/FR1998/001783
(87) Numéro de publication internationale: WO 1999/038511

(56) Documents cités:
- EP-A- 0 342 522
- EP-A1- 0 773 025
- WO-A-93/25204
- WO-A-96/01624
- WO-A-97/12581
- WO-A-98/19668
- WO-A-98/52564
- US-A- 5 385 739

## Description

La présente invention concerne une formulation galénique d'oméprazole sous forme de microgranules gastroprotégés ayant une stabilité dans le temps améliorée.

La présente invention s'étend en outre au procédé de fabrication desdits microgranules, et aux préparations pharmaceutiques les contenant.

L'oméprazole ou 5-méthoxy-2-[[(4-méthoxy-3,5-diméthyl-2-pyridinyl) méthyl]sulfinyl]-1-benzimidazole, est connu comme inhibiteur puissant de la sécrétion gastro-intestinale acide (brevet suédois n° 78 04231), et peut être utilisé pour le traitement des ulcères gastrique et duodénal.

Il est également connu que l'oméprazole se dégrade facilement en milieu acide et en milieu neutre. La demi-vie de dégradation de l'oméprazole est de dix minutes à un pH inférieur à 4, de dix-huit heures à un pH égal à 6,5 et d'environ 300 jours à un pH égala 11.

C'est pourquoi les formes galéniques d'oméprazole pour administration orale sont gastroprotégées pour que le principe actif atteigne l'intestin grêle sans être dégradé.

Au cours d'un stockage de longue durée dans des conditions d'utilisation habituelles (température de 25°C et degré d'humidité de l'ordre de 40-75 %), il a été observé que les formulations classiques ne sont pas stables dans le temps. On a observé la dégradation de l'oméprazole, l'apparition de produits de dégradation nocifs et la coloration de la formulation.

En effet, la stabilité de l'oméprazole est également affectée par l'humidité, la chaleur, la présence de solvants organiques même à l'état de traces, et la lumière à un moindre degré. Des solvants organiques sont généralement utilisés dans le procédé de fabrication des formulations d'oméprazole, ce que l'on souhaite éviter pour des raisons écologiques.

Afin d'améliorer la durée de stabilité au stockage des formulations gastroprotégées contenant de l'oméprazole ou un sel alcalin de l'oméprazole, le principe actif est souvent associé à un excipient tel que :
- une substance alcaline (voir demande de brevet EP-247 983), par exemple un sel de sodium, de potassium, de calcium ou d'aluminium, d'un acide organique, comme l'acide phosphorique, l'acide carbonique ou l'acide citrique.
- une substance anti-acide, par exemple un oxyde ou un hydroxyde d'aluminium, de magnésium ou de calcium,
- une substance organique tampon pharmaceutiquement acceptable, comme un acide aminé basique ou un de leurs sels, en particulier le trihydroxyméthylaminométhane,
- une substance inerte, comme du mannitol (voir brevet demande de EP-646 006) ou du dioxyde de titane (voir demande de brevet WO 96/37 195),
- un agent déshydratant lors du conditionnement final de la formulation.
- un tampon alcalin contenu dans des sous couches déposées séquentiellement entre les couches de principe actif, avec en outre un noyau alcalin (voir WO 98/19668).

Il est également connu d'améliorer la résistance acide de granules d'oméprazole destinés à être mis sous forme de comprimés, en utilisant des plastifiants dans la couche entérique (voir WO 96/01624).

Il a cependant été observé que la stabilité des formulations de l'art antérieur est insuffisante et le but de la présente invention est de fournir une formulation de microgranules d'oméprazole gastroprotégée, stable à la coloration, dont la stabilité au stockage de longue durée est améliorée, et qui présente, en outre, les propriétés thérapeutiques voulues, c'est-à-dire une certaine résistante à la dissolution en milieu acide, et une solubilité rapide en milieu neutre,.

L'objet de la présente invention est donc de fournir des microgranules d'oméprazole gastroprotégés présentant des profils de dissolution correspondant à l'application thérapeutique visée et qui sont avantageusement stables au cours du temps.

La présente invention concerne une nouvelle formulation d'oméprazole gastroprotégée contenant au moins une substance hydrophobe choisie pour augmenter la stabilité du principe actif tout en obtenant le profil de dissolution désiré. La Demanderesse a notamment
optimisé la composition d'une telle formulation en sélectionnant des combinaisons de plusieurs substances hydrophobes pour atteindre l'objectif de la présente invention.

Les microgranules d'oméprazole objet de la présente invention sont avantageusement dépourvus:
- de composés alcalins sous forme de sels,
- d'agents tensioactifs ioniques, comme le lauryl sulfate couramment utilisé pour stabiliser l'oméprazole, et
- de traces de solvants organiques.

Les microgranules d'oméprazole selon l'invention comportent chacun une couche active contenant le principe actif, et une couche externe de gastroprotection contenant un agent de gastroprotection et sont caractérisés en ce qu'ils contiennent au moins une substance hydrophobe choisie parmi les huiles siliconées.

On choisira des substances hydrophobes qui ne réagissent pas chimiquement avec l'oméprazole, qui peuvent être facilement mises en oeuvre lors de la formulation, qui soient compatibles avec les excipients utilisés et qui permettent d'obtenir les profils de dissolution et de libération voulus pour l'application thérapeutique visée.

Dans la couche active, la substance hydrophobe représente de préférence entre 5 et 40 % en poids de l'oméprazole.

Dans un mode de réalisation préféré, on enrobe avantageusement la couche active contenant l'oméprazole avec au moins une couche de protection.

Cette couche de protection peut contenir une substance diluante ou un agent d'enrobage associé à un plastifiant hydrophobe.

Dans la couche externe de gastroprotection, on pourra associer à l'agent de gastroprotection un agent hydrophobe, choisis de préférence parmi les glycérides.

Selon un mode de réalisation particulièrement avantageux, les microgranules selon l'invention utilisent une combinaison de différents agents hydrophobes permettant d'améliorer la stabilité de la formulation, dont une huile siliconée dans la couche active.

Selon un mode de réalisation préféré, les microgranules selon l'invention comportent:
- une couche de principe actif contenant l'oméprazole, un liant choisi parmi tous liants pharmaceutiquement acceptables, une huile siliconée et une substance solubilisante du principe actif,
- une première couche de protection contenant une ou plusieurs substances diluantes pharmaceutiquement acceptables, et un liant choisi parmi tous liants pharmaceutiquement acceptables,
- une deuxième couche de protection hydrophobe contenant un agent d'enrobage et un plastifiant hydrophobe,
- une couche de gastroprotection contenant un agent filmogène entérique, un plastifiant et une substance hydrophobe.

La couche de principe actif comporte avantageusement 5 à 15 %, par rapport au poids de principe actif, d'un tensio-actif non ionique choisi de préférence parmi les polysorbates (Montanox 80® ou Montane 20-60®).

La couche active comporte avantageusement un liant choisi parmi les liants pharmaceutiquement acceptables, en l'occurrence l'hydroxypropylméthylcellulose dont la proportion massique représente 30 à 50 % par rapport au poids de principe actif.

La première couche de protection comporte avantageusement une substance inerte choisie particulièrement parmi les diluants pharmaceutiquement acceptables dont le mannitol (qui est non hygroscopique) en proportion massique de 100 à 300 % et, préférentiellement, 200 % du poids du principe actif.

Cette couche comporte également un liant choisi parmi les liants pharmaceutiquement acceptables, avantageusement l'hydroxypropylméthylcellulose, en proportion de 10 à 30 % et, préférentiellement, 20 % du poids de mannitol.

Eventuellement, il peut être inclus dans cette couche de protection un lubrifiant choisi parmi les lubrifiants pharmaceutiquement acceptables, en l'occurrence le talc (qui est non hygroscopique) en proportion de 0 à 100 % du poids du principe actif.

La deuxième couche de protection est constituée d'un agent d'enrobage hydrosoluble choisi parmi les agents filmogènes pharmaceutiquement acceptables et avantageusement l'hydroxypropylméthylcellulose en proportion de 1 à 10% préférentiellement 5 %, du poids de microgranules obtenus après montage de la première couche de protection.

On utilisera avantageusement, dans la deuxième couche de protection, un plastifiant hydrophobe tel que le Myvacet® en proportion de 10 à 30 % du vernis sec de l'agent d'enrobage retenu.

Eventuellement, on utilisera un agent lubrifiant choisi parmi les lubrifiants pharmaceutiquement acceptables, avantageusement, le talc (qui est non hygroscopique), en proportion de 10 à 50 %, préférentiellement 15% du vernis sec de l'agent d'enrobage retenu.

La couche externe de gastroprotection contient un agent filmogène gastroprotecteur, avantageusement un copolymère d'acide méthacrylique, comme l'Eudragit L30D®, à raison de 15 à 30 %, préférentiellement 20 %, de dépôt sec de polymère par rapport à la masse de microgranules traitée.

Avantageusement, il sera inclus à l'agent filmogène gastroprotecteur une ou plusieurs substances hydrophobes choisies par les cires et huiles souvent utilisées dans l'industrie pharmaceutique, préférentiellement le Gélucire 50-13®, en proportion de 5 à 20 % du vernis sec de l'agent filmogène retenu.

On pourra éventuellement utiliser pour la couche exteme de gastroprotection un plastifiant choisi parmi les plastifiants pharmaceutiquement acceptables, préférentiellement le triéthylcitrate, représentant de 5 à 20 %, avantageusement 10 %, du poids de vernis sec dé l'agent filmogène retenu.

On utilisera, éventuellement, afin de renforcer la résistance contre l'humidité de la couche de gastroprotection, un agent lubrifiant choisi parmi les lubrifiants pharmaceutiquement acceptables, avantageusement le talc.

Selon un mode de réalisation préféré de la présente invention, la couche active est montée sur un noyau neutre constitué par exemple de saccharose et d'amidon, dont le diamètre est compris entre 700 et 900 microns.

Les microgranules selon l'invention auront, de préférence, une granulométrie comprise entre 0,5 et 3 mm, de préférence encore entre 0,7 et 2 mm.

La présente invention a également pour objet un procédé de préparation des microgranules selon l'invention. Ce procédé est caractérisé en ce qu'il est réalisé en milieu aqueux, sans utilisation d'aucun solvant organique.

Les microgranules décrits dans la présente invention seront obtenus par utilisation de tout équipement adéquat pour la préparation et l'enrobage de microgranules, bien connu de l'homme du métier et, en particulier, les équipements de type turbine conventionnelle, turbine perforée ou lit d'air fluidisé.

Selon un mode de réalisation préféré, les microgranules selon l'invention sont obtenus par montage sur noyau neutre, de préférence, en lit d'air fluidisé, par pulvérisations successives :
- d'une suspension aqueuse d'oméprazole et d'une huile siliconée,
- éventuellement d'une suspension aqueuse d'une substance diluante, et/ou
- d'une suspension aqueuse d'un agent d'enrobage et d'un plastifiant hydrophobe, et
- d'une suspension aqueuse de l'agent de gastroprotection dit également agent filmogène entérique.

Selon un mode de réalisation tout particulièrement apprécié, les microgranules selon l'invention sont montés sur noyau neutre en lit d'air fluidisé, par pulvérisations successives :
- d'une suspension aqueuse d'oméprazole et d'une huile siliconée,
- d'une suspension aqueuse de mannitol,
- d'une suspension aqueuse d'hydroxypropylcellulose, et
- d'une suspension aqueuse de l'agent de gastroprotection.

Chaque étape de pulvérisation est avantageusement suivie d'un tamisage et d'un séchage à une température inférieure à la température de fusion de chacun des composés entrant dans la constitution des microgranules à ladite étape.

Les microgranules obtenus selon ce procédé contiennent avantageusement moins de 1,5 %, de préférence 0,5 % en poids d'eau.

La présente invention a enfin pour objet les préparations pharmaceutiques contenant les microgranules selon l'invention susceptibles d'être obtenus par le procédé décrit précédemment, ces préparations seront avantageusement sous forme de gélules contenant 5 à 60 mg environ d'oméprazole.

D'autres caractéristiques et avantages de la présente invention apparaîtront à la lumière des exemples et des figures ci-après.

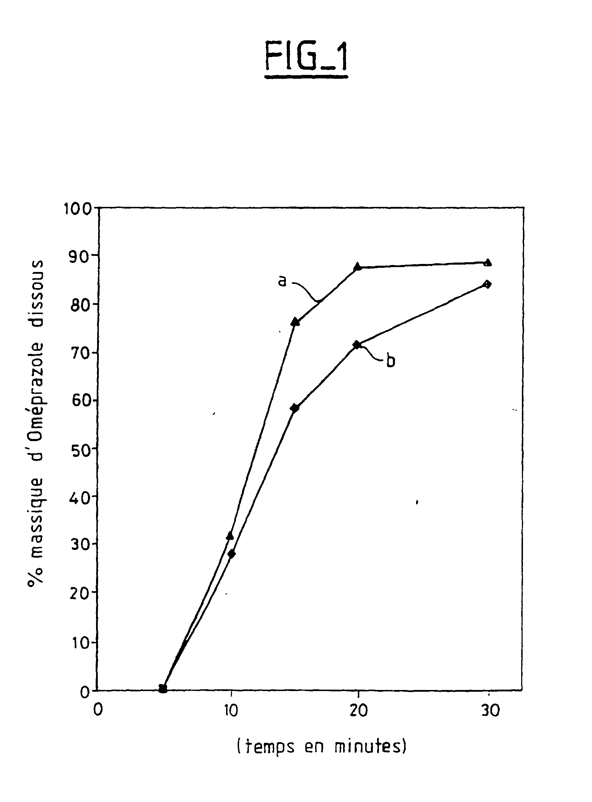

- La figure 1 représente la courbe de dissolution in vitro à pH 6,8 de gélules selon l'invention (courbe a) comparée à celle d'une formulation orale d'oméprazole de l'art antérieur (courbe b).

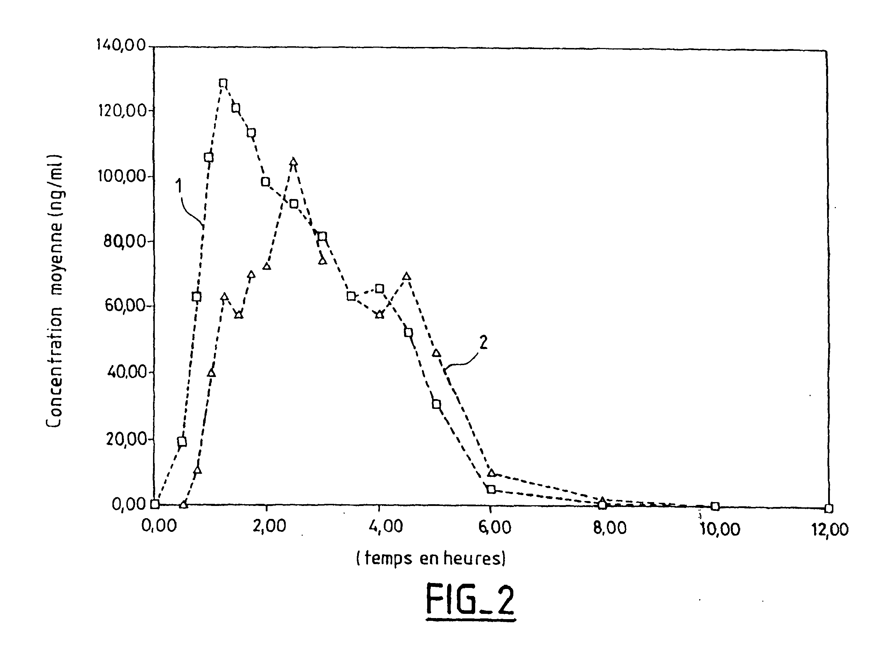

- La figure 2 représente la courbe de dissolution in vivo de gélules selon l'invention (courbe1) comparée à celle d'une formulation orale d'oméprazole de l'art antérieur (courbes2).
- la figure 3 représente l'évolution au cours du temps de la concentration plasmatique moyenne en oméprazole de 10 patients auxquels on a administré une formulation de l'invention (courbe A1), et de ces mêmes 10 patients auxquels on a administré une formulation de l'art antérieur (courbe B).
- la figure 4 représente l'évolution au cours du temps de la concentration plasmatique moyenne en oméprazole de 10 patients auxquels on a administré une formulation de l'invention (courbe A2), et de ces mêmes 10 patients auxquels on a administré une formulation de l'art antérieur (courbe C).

### EXEMPLE 1

On prépare des microgranules dans un appareil à lit d'air fluidisé de type OHLMAN.

### a) Montage du principe

On prépare une suspension du principe actif ayant la composition ci-dessous.

| **Composition de la suspension de principe actif** | **% massique** |
|---|---|
| Oméprazole | 14,9 |
| Pharmacoat 603® | 5,9 |
| Diméthicone | 4,2 |
| Polysorbate 80® | 1,5 |
| Eau purifiée | 73,5 |

L'eau purifiée est mise sous agitation et on ajoute successivement le Pharmacoat 603® (fabriqué par SEPPIC), le Polysorbate 80® (fabriqué par SEPPIC), le Diméthicone (fabriqué par LAMBERT et RIVIERE) et l'oméprazole.

L'agitation de la suspension est maintenue pendant tout le montage des Neutres 20® (fabriquées par NP PHARM) placées dans le lit d'air fluidisé.

Les Neutres® enrobées sont ensuite tamisées, et séchées pendant quatre heures à 50°C environ.

### b) Prémontage Pharmacoat®/Mannitol

On prépare une suspension de prémontage constituée de 4 % en poids de Pharmacoat 603®, 20 % en poids de Mannitol 25® (tous deux fabriqués par ROQUETTE) et 76 % d'eau purifiée.

Les neutres enrobées et séchées, obtenues précédemment sont pulvérisées avec cette suspension de prémontage.

Les neutres prémontées sont ensuite tamisées, puis séchées pendant une à quatre heures à 50°C environ.

### c) Prémontage Pharmacoat®/Myvacet®

On prépare une suspension de prémontage de composition suivante.

| **Composition de la suspension de prémontage** | **% massique** |
|---|---|
| Pharmacoat 603® | 7,14 |
| Myvacet 0,45® | 1,79 |
| Talc | 1,79 |
| Eau purifiée | 89,28 |

Cette étape de prémontage est effectuée dans les mêmes conditions que l'étape de prémontage Pharmacoat®/Mannitol.

Au cours des étapes a), b) et c), la température des granules est maintenue entre 26 et 28°C pendant la pulvérisation de la suspension.

### d) Enrobage Eudragit L30D®/Gelucire®

On prépare une suspension d'enrobage de composition suivante :

| **Composition de la suspension d'enrobage** | **% massique** |
|---|---|
| Eudragit L30D® | 54,64 |
| Triéthyl citrate | 1,64 |
| Gélucire 5013® | 1,64 |
| Eau purifiée | 42,08 |

en ajoutant le Gélucire® (fabriqué par GATTE FOSSE) fondu à 50°C.

Les microgranules enrobés sont ensuite tamisés et séchés à environ 45°C pendant quatre heures, puis lubrifiés avec du talc.

Les pertes à dessication des microgranules sont de l'ordre de 0,5 à 1 % après quinze minutes à 95°C, à la fin de chacune des étapes a) à d).

### EXEMPLE 2

On prépare des microgranules selon le procédé de l'exemple 1, afin d'obtenir les formulations A et B suivantes.

| **Composition** | **Pourcentage massique** | |
|---|---|---|
| | **Formulation A** | **Formulation B** |
| Neutres 20® | 47,5 | 36,2 |
| Oméprazole | 7,9 | 9,0 |
| Pharmacoat 630® | 9,1 | 11,2 |
| Diméthicone | 0,8 | 0,9 |
| Polysorbate 80® | 0,8 | 0,9 |
| Mannitol 25 | 12,0 | 20,3 |
| Myvacet 9.45V | 0,9 | 0,9 |
| Talc | 0,9 | 1,2 |
| Eudragit L30D® | 16,6 | 16,2 |
| Triéthylcitrate | 1,6 | 1,6 |
| Gélucire 5013® | 1,6 | 1,6 |

| | **Formulation A** | **Formulation B** |
|---|---|---|
| Teneur (mg/g) | 71 | 88 |
| Essai de gastrorésistance (Pourcentage massique) | | |
| au bout de 2 h à pH 1,2 | 1,8 | 1,4 |
| puis pendant 30 min à pH 6,8 | 88,3 | 86 |

Conformément à la Pharmacopée Européenne, les essais de dissolution in vitro sont réalisés avec un appareil à palette tournant à une vitesse de 100 tours/minute, dans 750 ml d'eau à 37°C ± 0,5°C et pH = 1,2, auxquels on ajoute au bout de deux heures, après avoir effectué la norme 250 ml d'une solution aqueuse de Na₃PO₄ à pH = 12,5 pour obtenir 1 l d'une solution à pH = 6,8.

### Etudes de stabilité

On évalue la stabilité de la formulation A non conditionnée au cours du temps dans des conditions de stockage réelles (i.e. à 25°C et 40% d'humidité relative).

| **Formulation A** | **t = O** | **t = 11 mois** |
|---|---|---|
| Essai de gastrorésistance (%) | | |
| au bout de 2 h à pH 1,2 | 4,6 | 1,4 |
| puis pendant 30 min à pH 6,8 | 96,3 | 94,1 |
| Teneur en impuretés (%) | 0,4 | 0,4 |
| Couleur | blanchâtre | blanchâtre |

On conditionne les microgranules A et B préparés dans l'exemple 2 dans des gélules de taille 2, respectivement notées G_{A} et G_{B}.

On réalise une première étude dans des conditions de vieillissement accéléré, selon un essai normalisé ICH (à 40°C et 75 % d'humidité relative).
Les essais de stabilité en fonction du temps des gélules G_{A} et G_{B} sont réalisés en plaçant les gélules dans des flacons de polyéthylène opaques.

Les impuretés sont dosées par spectrométrie U.V. après séparation par chromatographie liquide haute performance.

Les essais de gastrorésistance sont réalisés dans les mêmes conditions que dans l'exemple 2 et doivent vérifier la norme suivante pour être jugés positifs

| | | |
|---|---|---|
| 10 % ou moins | après deux heures | à pH 1,2 |
| et plus de 75 % | après trente minutes | à pH 6,8. |

Le dosage en principe actif et la teneur en eau sont respectivement effectués selon les normes USP <905> et USP <921>.

Les résultats de stabilité des gélules G_{A} et G_{B} en condition de vieillissement accéléré sont résumés respectivement dans les tableaux 1 et 2.

**TABLEAU 1**

| **G_{A}** | **t = O** | **1 mois** | **2 mois** | **3 mois** |
|---|---|---|---|---|
| Dosage (mg/gélule) | 14 | 14,2 | 14,2 | 11,8 |
| Essai de gastrorésistance (%) | | | | |
| au bout de 2 h à pH 1,2 | 4,6 | 0,0 | 2,0 | 3,3 |
| puis pendant 30 min à pH 6,8 | 96,3 | 90,2 | 86,3 | 94,0 |
| Teneur en eau (%) | 1,4 | 2,0 | 1,8 | 1,9 |
| Teneur en impuretés (%) | 0,4 | 1,0 | 2,7 | 10,2 |
| Couleurs | blanchâtre | blanchâtre | gris | gris |

**TABLEAU 2**

| **G_{B}** | **t = O** | **1 mois** | **2 mois** |
|---|---|---|---|
| Dosage (mg/gélule) | 20 | 19,7 | 19,9 |
| Essai de gastrorésistance (%) | | | |
| au bout de 2 h à pH 1,2 | 2 | 2,4 | 2,5 |
| puis pendant 30 min à pH 6,8 | 92,4 | 91,9 | 92,4 |
| Teneur en eau (%) | 0,8 | 1,4 | 1,1 |
| Teneur en impuretés (%) | 0,3 | 4,5 | 4,8 |
| Couleur | Blanchâtre | Blanchâtre | Blanchâtre et reflets gris |

On réalise ensuite une étude comparée de la stabilité des microgranules G_{A} et d'une formulation de l'art antérieur, toujours dans des conditions de vieillissement accéléré.

Le tableau 3 montre la stabilité accrue des microgranules selon la présente invention par rapport à une formulation de l'art antérieur commercialisée par la Société ASTRA sous le nom de marque Mopral® (brevet EP-247 983).

**TABLEAU 3**

| | **t = O** | **1 mois** | **2 mois** | **3 mois** |
|---|---|---|---|---|
| Teneur en impuretés (%) | | | | |
| Mopral® | - | 1,06 | 22,6 | 38,17 |
| G_{A} | 0,4 | 1,0 | 2,7 | 10,2 |
| Couleurs | | | | |
| Mopral® | - | marron | marron | marron |
| G_{A} | blanchâtre | blanchâtre | gris | gris |

### ESSAIS CLINIQUES

Ces essais visent à vérifier que les formulations selon l'invention n'occasionnent pas de perte de biodisponibilité.
• On réalise une première étude en comparant les paramètres de biodisponibilité d'une formulation selon l'invention notée A1 et d'une formulation de l'art antérieur notée B commercialisée sous le nom de marque Losec® par la société ASTRA.
Cette étude randomisée est menée sur 10 patients auxquels on administre une dose unique de 20 mg de microgranules selon l'invention.
La concentration plasmatique en oméprazole est suivie pendant les huit heures suivant l'administration.
Après un repos de 7 jours, ces mêmes dix patients reçoivent une dose unique de 20 mg d'une formulation de l'art B. De la même façon, la concentration plasmatique en oméprazole est mesurée régulièrement pendant les huit heures suivant l'administration.

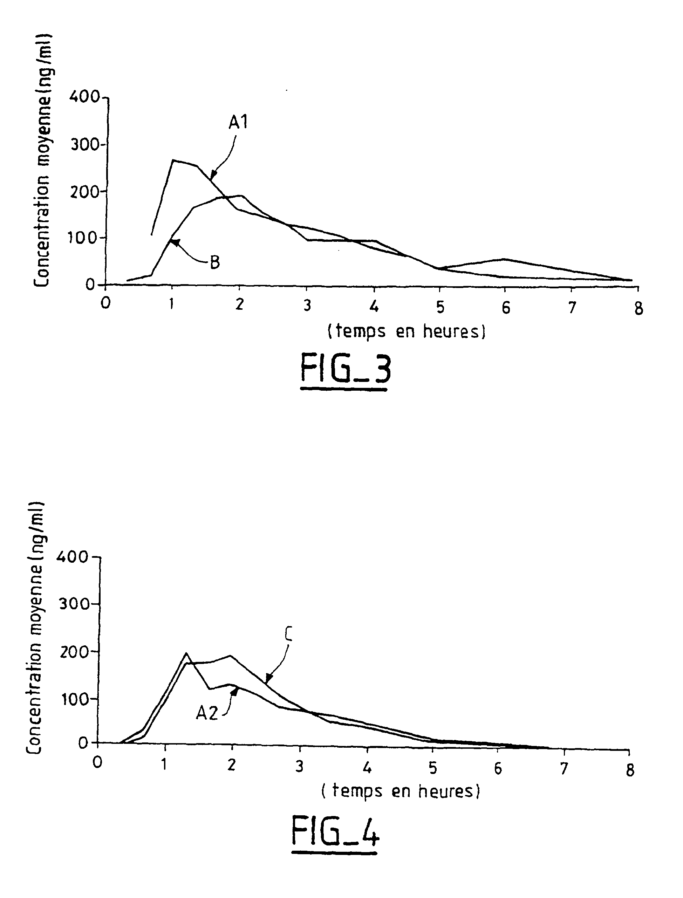

La figure 3 représente l'évolution de la concentration plasmatique moyenne en oméprazole (mesurée sur les 10 patients) calculée pour A1 et puis pour B.
Le tableau 4 donne la valeur moyenne des principaux paramètres de biodisponibilité correspondant aux deux courbes A1 et B.

**TABLEAU 4**

| **Traitement** | **AUCo-t (ng/ml)** | **AUCinf (ng/ml)** | **Cmax (ng/ml)** | **Tmax (h)** | **Kel** | **T1/2 (h)** |
|---|---|---|---|---|---|---|
| **A1** | 544,7 | 618,6 | 336,3 | 2,68 | 0,097 | 0,76 |
| Coefficient de variation | (48 %) | (42 %) | (37 %) | (63 %) | (31 %) | (29 %) |
| **B** | 517,5 | 554,1 | 291,1 | 2,26 | 1,00 | 0,75 |
| Coefficient de variation | (57 %) | (55 %) | (42 %) | (44 %) | (29 %) | (32 %) |
| Rapport **A1/B** | 98 % | 94 % | 83 % | - | - | - |

• Une deuxième étude est réalisée dans les mêmes conditions que précédemment en administrant une formulation selon l'invention notée A2 puis une formulation de l'art antérieur notée C commercialisée sous la marque Prilosec® par la Société MERCK.
La figure 4 représente l'évolution au cours du temps de la concentration plasmatique moyenne en oméprazole des 10 patients mesurée pour A2 et C.
Le tableau 5 donne la valeur moyenne des principaux paramètres de biodisponibilité correspondant aux deux courbes A2 et C.

**TABLEAU 5**

| **Traitement** | **AUCₒ₋ₜ (ng/ml)** | **AUC_{inf} (ng/ml)** | **Cₘₐₓ (ng/ml)** | **Tₘₐₓ (h)** | **Kₑₗ** | **T_{1/2} (h)** |
|---|---|---|---|---|---|---|
| **A2** | 423,2 | 431,9 | 274,6 | 2,38 | 1,11 | 0,63 |
| Coefficient de variation | (48 %) | (56 %) | (51 %) | (46 %) | (13 %) | (14 %) |
| **C** | 429,0 | 459,8 | 283,6 | 1,95 | 1,07 | 0,67 |
| Coefficient de variation | (41 %) | (40 %) | (51 %) | (50 %) | (21 %) | (25 %) |
| Rapport **A2/C** | 117% | 112% | 107% | - | - | - |

## Revendications

1. Microgranules d'oméprazole comportant chacun une couche active contenant le principe actif, et une couche externe de gastroprotection contenant un agent de gastroprotection, **caractérisés en ce que** les microgranules sont dépourvus de composés alcalins sous forme de sels et que la couche active contient une substance hydrophobe choisie parmi les huiles siliconées.

2. Microgranules selon la revendication 1, **caractérisés en ce que** la substance hydrophobe contenue dans la couche active représente 5 à 40 % en poids d'oméprazole.

3. Microgranules selon la revendication 1 ou 2, **caractérisés en ce qu'**on enrobe la couche active avec au moins une couche de protection.

4. Microgranules selon l'une des revendications 1 à 3, **caractérisés en ce que** la couche de protection contient une substance diluante, ou un agent d'enrobage associé à un plastifiant hydrophobe.

5. Microgranules selon l'une des revendications 1 à 4, **caractérisés en ce que** dans la couche externe de gastroprotection, on associe à l'agent de gastroprotection un agent hydrophobe.

6. Microgranules selon l'une des revendications 1 à 5, **caractérisés en ce que** chaque microgranule contient :
• une couche de principe actif contenant l'oméprazole, un liant choisi parmi tous liants pharmaceutiquement acceptables, ladite substance hydrophobe contenue dans la couche active et une substance solubilisante du principe actif,
• une première couche de protection contenant une ou plusieurs substances diluantes pharmaceutiquement acceptables et un liant,
• une deuxième couche de protection hydrophobe contenant un agent d'enrobage et un plastifiant hydrophobe,
• une couche de gastroprotection contenant un agent filmogène entérique, un plastifiant et une substance hydrophobe.

7. Microgranules selon la revendication 6, **caractérisés en ce que** la substance solubilisante est un tensio-actif non ionique choisi parmi les polysorbates.

8. Microgranules selon l'une des revendications 6 à 7, **caractérisés en ce que** le liant est l'hydroxypropylméthylcellulose.

9. Microgranules selon l'une des revendications 6 à 8, **caractérisés en ce que** la première couche de protection comporte du mannitol comme substance diluante.

10. Microgranules selon l'une des revendications 6 à 9, **caractérisés en ce que** la deuxième couche de protection est constituée d'un agent d'enrobage comme l'hydroxypropylméthylcellulose et d'un plastifiant hydrophobe comme le Myvacet®.

11. Microgranules selon l'une des revendications 6 à 10, **caractérisés en ce que** la substance hydrophobe contenue dans la couche de gastroprotection est choisie parmi les glycérides.

12. Microgranules selon l'une des revendications précédentes, **caractérisés en ce que** la couche active est montée sur un noyau neutre, et que la granulométrie des microgranules est comprise entre 0,5 et 3 mm.

13. Procédé de préparation des microgranules selon l'une des revendications précédentes, **caractérisé en ce qu'**il est réalisé en milieu aqueux.

14. Procédé selon la revendication 13, **caractérisé en ce qu'**on procède à un montage en lit d'air fluidisé.

15. Préparations pharmaceutiques contenant les microgranules selon l'une des revendications 1 à 12, ou obtenus selon le procédé de l'une des revendications 13 ou 14, contenant 5 à 60 mg environ d'oméprazole.

## Claims

1. Omeprazole microgranules, each comprising an active layer comprising the active principle and an external gastroprotective layer comprising a gastroprotection agent, **characterized in that** the microgranules are devoid of alkaline compounds in the form of salts and **in that** the active layer comprises a hydrophobic substance chosen from silicone oils.

2. Microgranules according to Claim 1, **characterized in that** the hydrophobic substance present in the active layer represents 5 to 40% by weight of the omeprazole.

3. Microgranules according to Claim 1 or 2, **characterized in that** the active layer is coated with at least one protective layer.

4. Microgranules according to one of Claims 1 to 3, **characterized in that** the protective layer comprises a diluent substance or a coating agent in combination with a hydrophobic plasticizer.

5. Microgranules according to one of Claims 1 to 4, **characterized in that**, in the external gastroprotective layer, a hydrophobic agent is used in combination with the gastroprotection agent.

6. Microgranules according to one of Claims 1 to 5, **characterized in that** each microgranule comprises
• a layer of active principle comprising omeprazole, a binder chosen from any pharmaceutically acceptable binder, the said hydrophobic substance present in the active layer and a substance which dissolves the active principle,
• a first protective layer comprising one or more pharmaceutically acceptable diluent substances and a binder,
• a second hydrophobic protective layer comprising a coating agent and a hydrophobic plasticizer,
• a gastroprotective layer comprising an enteric film-forming agent, a plasticizer and a hydrophobic substance.

7. Microgranules according to Claim 6, **characterized in that** the solubilizing substance is a non-ionic surfactant chosen from polysorbates.

8. Microgranules according to either of Claims 6 and 7, **characterized in that** the binder is hydroxypropylmethylcellulose.

9. Microgranules according to one of Claims 6 to 8, **characterized in that** the first protective layer comprises mannitol as diluent substance.

10. Microgranules according to one of Claims 6 to 9, **characterized in that** the second protective layer is composed of a coating agent, such as hydroxypropylmethylcellulose, and of a hydrophobic plasticizer, such as Myvacet^{®}.

11. Microgranules according to one of Claims 6 to 10, **characterized in that** the hydrophobic substance comprised within the gastroprotective layer is chosen from glycerides.

12. Microgranules according to one of the preceding claims, **characterized in that** the active layer is coated on a neutral core and **in that** the particle size of the microgranules is between 0.5 and 3 mm.

13. Process for the preparation of the microgranules according to one of the preceding claims, **characterized in that** it is carried out in aqueous medium.

14. Process according to Claim 13, **characterized in that** a coating operation is carried out in a fluidized air bed.

15. Pharmaceutical preparations comprising the microgranules according to one of Claims 1 to 12, or obtained according to the process of either of Claims 13 and 14, comprising 5 to 60 mg approximately of omeprazole.

## Patentansprüche

1. Omeprazol-Mikrokörner, die jeweils eine aktive, den Wirkstoff enthaltende Schicht und eine äußere, ein Schutzmittel gegen den Magensaft enthaltende Magensaftschutzschicht umfassen, **dadurch gekennzeichnet, dass** die Mikrokörner keine alkalischen Verbindungen in Form von Salzen aufweisen und dass die aktive Schicht eine hydrophobe Substanz enthält, die aus siliconhaltigen Ölen ausgewählt ist.

2. Mikrokörner nach Anspruch 1, **dadurch gekennzeichnet, dass** die aktive Schicht eine hydrophobe Substanz enthält, die 5 bis 40 Gew.-% des Omeprazols ausmacht.

3. Mikrokörner nach Anspruch 1 oder 2, **dadurch gekennzeichnet, dass** die aktive Schicht mit wenigstens einer Schutzschicht umhüllt ist.

4. Mikrokörner nach einem der Ansprüche 1 bis 3, **dadurch gekennzeichnet, dass** die Schutzschicht eine Verdünnungssubstanz oder ein Umhüllungsmittel enthält, das mit einem hydrophoben Weichmacher kombiniert ist.

5. Mikrokörner nach einem der Ansprüche 1 bis 4, **dadurch gekennzeichnet, dass** in der äußeren Magensaftschutzschicht ein hydrophobes Mittel mit dem Schutzmittel gegen den Magensaft kombiniert ist.

6. Mikrokörner nach einem der Ansprüche 1 bis 5, **dadurch gekennzeichnet, dass** jedes Mikrokorn enthält:
• eine Wirkstoffschicht, die Omeprazol, ein Bindemittel, das aus jeglichen pharmazeutisch verträglichen Bindemitteln ausgewählt ist, eine hydrophobe Substanz und eine den Wirkstoff solubilisierende Substanz enthält,
• eine erste Schutzschicht, die eine oder mehrere pharmazeutisch verträgliche Verdünnungssubstanzen und ein Bindemittel enthält,
• eine zweite hydrophobe Schutzschicht, die ein Umhüllungsmittel und einen hydrophoben Weichmacher enthält,
• eine Magensaftschutzschicht, die ein enterisches filmbildendes Mittel, einen Weichmacher und eine hydrophobe Substanz enthält.

7. Mikrokörner nach Anspruch 6, **dadurch gekennzeichnet, dass** die solubilisierende Substanz ein nichtionisches grenzflächenaktives Mittel ist, das aus Polysorbaten ausgewählt ist.

8. Mikrokörner nach einem der Ansprüche 6 bis 7, **dadurch gekennzeichnet, dass** das Bindemittel Hydroxypropylmethylcellulose ist.

9. Mikrokörner nach einem der Ansprüche 6 bis 8, **dadurch gekennzeichnet, dass** die erste Schutzschicht Mannitol als Verdünnungssubstanz umfasst.

10. Mikrokörner nach einem der Ansprüche 6 bis 9, **dadurch gekennzeichnet, dass** die zweite Schutzschicht aus einem Umhüllungsmittel, wie Hydroxypropylmethylcellulose, und einem hydrophoben Weichmacher, wie Myvacet®, gebildet ist.

11. Mikrokörner nach einem der Ansprüche 6 bis 10, **dadurch gekennzeichnet, dass** die in der Magensaftschutzschicht enthaltene hydrophobe Substanz aus Glyceriden ausgewählt ist.

12. Mikrokörner nach einem der vorangegangenen Ansprüche, **dadurch gekennzeichnet, dass** die aktive Schicht auf einen neutralen Kern aufgebracht ist und dass die Korngrößenverteilung der Mikrokörner zwischen 0,5 und 3 mm einschließlich liegt.

13. Verfahren zur Herstellung der Mikrokörner nach einem der vorangegangenen Ansprüche, **dadurch gekennzeichnet, dass** es in wässrigem Medium ausgeführt wird.

14. Verfahren nach Anspruch 13, **dadurch gekennzeichnet, dass** man einen Aufbau in einer luftdurchströmten Wirbelschicht vornimmt.

15. Pharmazeutische Zubereitungen, die die Mikrokörner nach einem der Ansprüche 1 bis 12 oder erhalten nach dem Verfahren von einem der Ansprüche 13 oder 14 enthalten, welche ungefähr 5 bis 60 mg Omeprazol enthalten.
